Europäisches Patentamt

European Patent Office

Office européen des brevets

(19)

(11) Veröffentlichungsnummer: **0 319 933**
**A2**

## (12) EUROPÄISCHE PATENTANMELDUNG

(21) Anmeldenummer: 88120393.9

(22) Anmeldetag: 07.12.88

(51) Int. Cl.⁴: **A61M 16/00**

(30) Priorität: 08.12.87 DE 3741454

(43) Veröffentlichungstag der Anmeldung:
14.06.89 Patentblatt 89/24

(84) Benannte Vertragsstaaten:
**DE FR GB NL**

(71) Anmelder: **Drägerwerk Aktiengesellschaft**
**Moislinger Allee 53-55**
**D-2400 Lübeck 1(DE)**

(72) Erfinder: **Frank, Helge**
**Wickedestrasse 6**
**D-2400 Lübeck(DE)**

(54) **Kalibrierventil für Gassensoren.**

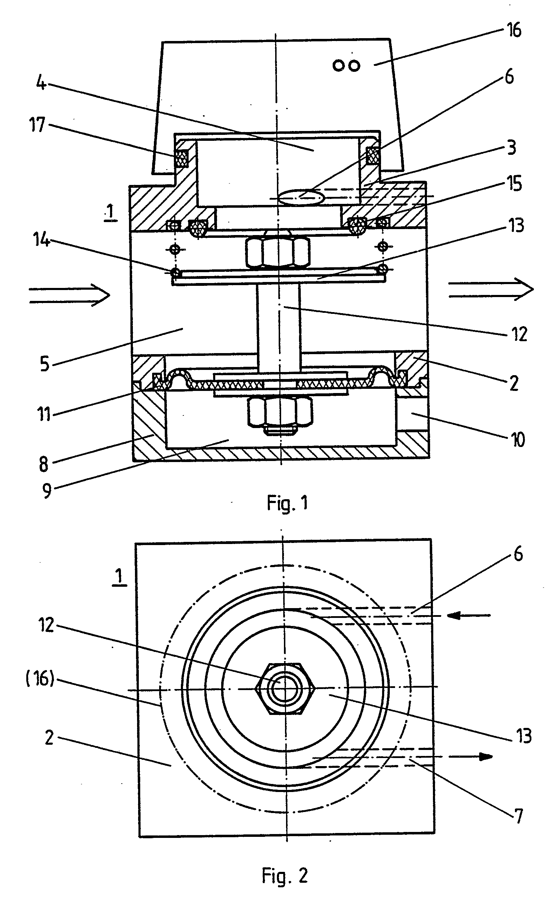

(57) Ein Kalibrierventil für Gassensoren, insbesondere für einen Sauerstoffsensor in der Atemgasführung eines Beatmungsgerätes soll so aufgebaut sein, daß die Kalibrierung des Sensors ohne wesentliche Veränderung der Strömung durch die Ventildurchgangsöffnung erfolgen kann. Dies wird erfindungsgemäß dadurch erreicht, daß in Verbindung mit der Ventildurchgangsöffnung (5) ein durch ein Ventilelement (12, 13) absperrbarer, mit einem Spülgas- bzw. Kalibriergaseinlaß (6) und -auslaß (7) versehener Sensorteilraum (4) vorgesehen ist, und daß das Ventilelement (12,13) durch einen Steuerantrieb (11) in zwei Endlagen bewegbar ist, wobei der Sensorteilraum (4) in ger einen Endlage bei geschlossenem Spülgas-bzw. Kalibriergaseinlaß (6) und -auslaß (7) in Strömungsverbindung mit der Ventildurchgangsöffnung (5) steht, während er in der anderen Endlage durch das Ventilelement (12,13) von der Ventildurchgangsöffnung (5) abgesperrt ist (Fig. 1).

Fig. 1

EP 0 319 933 A2

## Kalibrierventil für Gassensoren

Die Erfindung betrifft ein Kalibrierventil für Gassensoren, insbesondere für einen Sauerstoffsensor in der Atemgasführung eines Beatmungsgerätes.

Bei Beatmungsgeräten kommt einer fortgesetzten Überwachung des Sauerstoffgehaltes im Atemgas besondere Bedeutung zu. Ein Sauerstoffsensor in der Atemgasführung eines Narkosegerätes ist u.a. in der US-PS 4 127 121 beschrieben.

Derartige Sauerstoffsensoren werden ferner bei Langzeitbeatmungsgeräten in der Intensivpflege benutzt. Dabei ergibt sich die Notwendigkeit, die Sensoren nach einer bestimmten Betriebsdauer neu zu kalibrieren. Hierzu muß der Sensor einer Atmosphäre von bekannter Sauerstoffkonzentration ausgesetzt werden. Dies wird im allgemeinen dadurch erreicht, daß die sauerstoffempfindliche Meßoberfläche des Sensors mit reinem Sauerstoff (100 Vol.% $O_2$) bzw. mit Luft (21 Vol.% $O_2$) gespült wird.

Der Sauerstoffsensor eines Beatmungsgerätes befindet sich in direktem Kontakt mit dem Atemgasstrom. Wird der Sensor mit Kalibriergas gespült, ohne vom Atemgasstrom abgetrennt zu werden, ergibt sich u.U. eine unerwünschte Beeinflussung in der Zusammensetzung des Atemgasstromes. Wird die Kalibrierung, die etwa 2 Minuten in Anspruch nimmt, unmittelbar vor einer nachfolgenden Blutgasanalyse des Patienten durchgeführt, so können durch den dem Atemgas beigemischten Anteil des Kalibriergases erheblich verfälschte Blutgaswerte ermittelt werden. Eine auf diesen Werten basierende Neueinstellung des Beatmungsgerätes kann zur Fehlbeatmung des Patienten führen. Besonders nachteilig erscheint dieser Effekt in der Früh- und Neugeborenenbeatmung, da wegen der an sich geringeren Strömungsmenge der Einfluß des Kalibriergasanteils größer sein kann als der des eigentlichen Atemgasgemisches. Es ist daher erwünscht, die Kalibrierung des Gassensors ohne Beeinträchtigung der Atemgasströmung durchzuführen.

Zum Stande der Technik gehört nach der DE-OS 33 15 509 ein Elektroden-Eichgerät für zur Flüssigkeitsuntersuchung benutzte Fühlelektroden, wobei ein bewegliches Ventil vorgesehen ist, welches während der Strömung der zu untersuchenden Flüssigkeit einen Behälter mit Eichlösung absperrt und den Zutritt der Eichlösung zu der Fühlelektrode nach Beendigung der Strömung der zu untersuchenden Flüssigkeit ermöglicht. Damit läßt sich bei unterbrochener Strömung der zu untersuchenden Flüssigkeit eine Eichung der Fühlelektrode ausführen.

Die Erfindung geht von der Aufgabenstellung aus, ein Kalibrierventil für Gassensoren, insbesondere für Sauerstoffsensoren in der Atemgasführung eines Beatmungsgerätes zu schaffen, welches die Kalibrierung des Sensors ohne Veränderung (d.h. Strömungsbehinderung oder Zusammensetzungsänderung) einer weiterhin aufrecht erhaltenen Hauptgasströmung, insbesondere in der Atemgasführung ermöglicht.

Diese Aufgabenstellung wird gemäß der Erfindung dadurch gelöst, daß in Verbindung mit der Ventildurchgangsöffnung ein durch ein Ventilelement absperrbarer mit einem Spül- bzw. Kalibriergaseinlaß und -auslaß versehener Sensorteilraum vorgesehen ist, und daß das Ventilelement durch einen Steuerantrieb in zwei Endlagen bewegbar ist, wobei der Sensorteilraum in der einen Endlage bei geschlossenem Spülgas- bzw. Kalibriergaseinlaß und -auslaß in Strömungsverbindung mit der Ventildurchgangsöffnung steht, während er in der anderen Endlage durch das Ventilelement von der Ventildurchgangsöffnung abgesperrt ist. Mit einem solchen Kalibrierventil wird somit in geöffnetem Zustand der Gas- bzw. Sauerstoffsensor in direkten Kontakt mit dem Hauptgasstrom bzw. Atemgasstrom gebracht und in geschlossenem Zustand vom Hauptgasstrom abgetrennt, so daß die Spülung des Sensors mit Kalibriergas ermöglicht wird. In offenem und geschlossenem Zustand kann die Hauptgasströmung bzw. Atemgasströmung das Ventil frei passieren.

In weiterer Ausbildung der Erfindung kann es zweckmäßig sein, die Steuerung von Spülgas- bzw. Kalibriergaseinlaß und -auslaß mit dem Steuerantrieb des Ventilelementes zu koppeln. Das Ventilelement kann zweckmäßig mit einer einen Druckraum abschließenden Schaltmembran oder mit einem elektromagnetischen Steuerantrieb verbunden sein. Spülgas- bzw. Kalibriergaseinlaß und -auslaß müssen über entsprechende Absperrelemente so gesteuert werden, daß bei mit der Hauptgasströmung verbundenem Sensorteilraum der Spülgas- bzw. Kalibriergaseinlaß und -auslaß abgesperrt werden. Bei von der Hauptgasströmung getrenntem Sensorteilraum müssen dagegen der Spülgas- bzw. Kalibriergaseinlaß zur Zufuhr von Spül- bzw. Kalibriergas in den abgesperrten Sensorteilraum und zur Ableitung des Gases in den Umgebungsraum geöffnet werden. Diese Verbindung zwischen der Ein- und Auslaßsteuerung für den Sensorteilraum kann vorteilhaft mechanisch von der Bewegung des Ventilelementes abgeleitet werden, wobei diese Bewegung entsprechende im Kalibrierventil zusätzlich angeordnete Absperrelemente steuert, oder sie kann durch elektrisch gesteuerte Absperrelemente in Verbindung mit dem Steuerantrieb erfolgen.

Der Sensorteilraum wird zweckmäßig durch einen seitlichen Ansatz des Ventilgehäuses gebildet. Dabei kann dieser Gehäuseansatz vorteilhaft ein Steckansatz zum Aufstecken des auswechselbaren Sensors sein.

Eine günstige Ausführungsform kann vorsehen, daß das Ventilelement eine gegen eine Ringdichtung des Gehäuseansatzes anliegende Platte ist, welche mit einer Öffnungsvorspannungsfeder abgestützt ist.

Wenn die Steuerung von Einlaß und Auslaß des Spül- bzw. Kalibriergases im Sensorteilraum nicht unmittelbar durch mechanische Kopplung der Absperrelemente mit der Bewegung des Ventiltellers erfolgt, kann eine Schaltungsanordnung vorteilhaft sein, bei der ein 2/2 Wege-Magnetventil vorgesehen ist, welches die Schaltmembran des Kalibrierventils und gleichzeitig die Spülgaszuführung zum Zufluß von Spülgas in den Einlaß des Sensorteilraums schaltet, während der Auslaß des Sensorteilraums durch einen mit dem 2/2 Wege-Magnetventil verbundenen pneumatischen Schalter nach der Atmosphäre hin geöffnet wird.

Ein weiterer Vorteil kann gegebenenfalls dadurch erreicht werden, daß in der Gaszuleitung zum Spül- bzw. Kalibriergaseinlaß und in der Zuführungsleitung zum Druckraum der Schaltmembran durchflußbegrenzende Drosselstellen eingeschaltet sind. Die Drosselstelle vor dem Spül- bzw. Kalibriergaseinlaß ermöglicht die Dosierung der Menge des zugeführten Spül- bzw. Kalibriergases, während die Drosselstelle in der Zuführungsleitung zum Druckraum der Schaltmembran den Gasstrom begrenzt, der beim Reißen der Schaltmembran in die Hauptgasströmung, insbesondere in die Atemgasströmung zum Patienten, gelangen könnte.

Die Erfindung wird nachfolgend unter Bezug auf die Zeichnung näher erläutert; es zeigen:

Fig. 1 einen Längsschnitt durch ein Kalibrierventil für einen Sauerstoffsensor,

Fig. 2 eine Draufsicht auf das Kalibrierventil nach Fig. 1,

Fig. 3 eine Schaltungsanordnung zur Benutzung in Verbindung mit einem Kalibrierventil nach Fig. 1 und 2.

In den Figuren 1 und 2 ist ein Kalibrierventil 1 dargestellt, welches an einem Ventilgehäuse 2 einen seitlichen Ansatz 3 zur Bildung eines Sensorteilraums 4 aufweist. Der Sensorteilraum 4 mündet in die Ventildurchgangsöffnung 5.

In den Sensorteilraum münden in seitlicher Einführung ein Spülgas- bzw. Kalibriergaseinlaß 6 und ein Spülgas-bzw. Kalibriergasauslaß 7 (vergl. Fig. 2).

An das Ventilgehäuse 2 ist ein Ansatzteil 8 mit einer Einlaßöffnung 10 angesetzt, welcher einen Druckraum 9 bildet.

Der Druckraum 9 ist gegenüber der Ventildurchgangsöffnung 5 mit einr Schaltmembran 11 abgeschlossen, die mit einem aus einer Ventilstange 12 und einer Ventilplatte 13 zusammmengesetzten Ventilelement in Verbindung steht. Die Ventilplatte 13 ist durch eine Vorspannöffnungsfeder 14, die einseitig gehäusefest abgestützt ist, in Öffnungsrichtung vorgespannt. Als Dichtsitz der Ventilplatte 13 dient eine im Ventilgehäuse angeordnete Schnurringdichtung 15.

Der seitliche Gehäuseansatz 3 ist als Steckansatz zum Aufstecken eines Sauerstoffsensors 16 ausgebildet. Die Abdichtung der Steckverbindung erfolgt durch eine weitere Schnurringdichtung 17.

Das in Fig.1 in Öffnungsendlage gezeigte Kalibrierventil, wobei der Sensorteilraum 4 mit der Ventildurchlaßöffnung 5 kommuniziert, kann durch Druckaufbau im Druckraum 9 in seine Absperrendlage gebracht werden, in der der Sensorteilraum 4 von der Ventildurchgangsöffnung 5 abgesperrt ist. Zur Absperrung bzw. Freigabe des Ein-und Auslasses 6,7 für Spülgas- bzw. Kalibriergas sind zusätzliche steuerbare Absperrelemente erforderlich, die außerhalb des eigentlichen Kalibrierventils 1 angeordnet sein können.

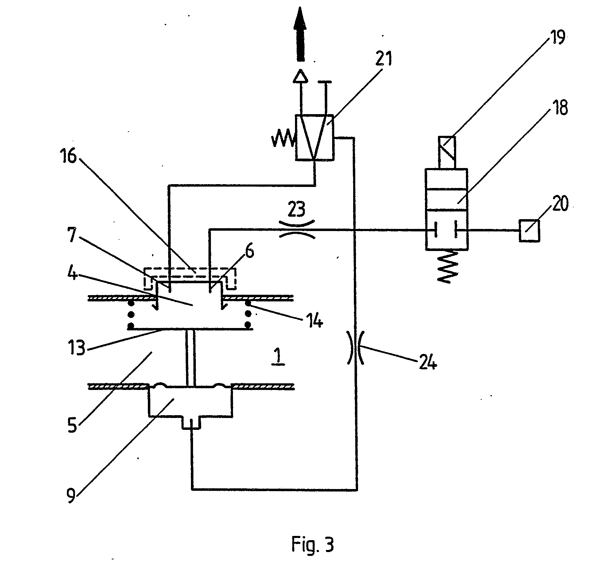

Eine Schaltungsanordnung zur Benutzung mit einem oben angegebenen Kalibrierventil 1 ist in Fig. 3 erläutert. Hierbei werden als Kalibriergase atmosphärische Luft und Sauerstoff verwendet. Ein 2/2 Wege-Magnetventil 18, welches eine Betätigungsspule 19 zur Auslösung des Schalt- bzw. Kalibriervorgangs aufweist, ist eingangsseitig mit einer Druckluft-oder Sauerstoffquelle 20 und ausgangsseitig mit dem Spül- bzw. Kalibriergaseinlaß 6 des Kalibrierventils 1, ferner mit dem Druckraum 9 und mit einem pneumatischen Schalter 21 verbunden. Der pneumatische Schalter 21 steuert den Auslaß 7 für Spül- bzw. Kalibriergas.

In der Zuführungsleitung zum Einlaß 6 ist eine Drosselstelle 23 und in der Zuführungsleitung zum Druckraum 9 eine weitere Drosselstelle 24 vorgesehen.

Der aufgesteckte Sauerstoffsensor 16 ist gestrichelt angedeutet.

Zur Auslösung des Kalibriervorganges wird die Betätigungsspule 19 des 2/2 Wege-Magnetventils 18 betätigt. Dadurch wird dem Druckraum 9 Druckgas zugeführt, so daß die Ventilplatte 13 in die Absperrendlage gelangt.Außerdem strömt Luft oder Sauerstoff atmosphärischen Drucks über die Drosselstelle 23 in den Einlaß 6 des Sensorteilraums 4. Gleichzeitig wird der Druckschalter 21 durch die Druckgaszuführung geöffnet und ermöglicht den Austritt des Spül- bzw. Kalibriergases aus dem Auslaß 7.

Damit ist der Sensorteilraum 4 von der Ventildurchgangsöffnung 5 abgesperrt und der Sensor kann nach entsprechendem Spülvorgang mit Luft oder mit reinem Sauerstoff kalibriert werden.

Nach Abschluß des Kalibriervorgangs wird das 2/2 Wege-Magnetventil 18 durch Abschalten der Betätigungsspule 19 in die Öffnungsstellung gesteuert. Dadurch werden Ein- und Auslaß 6,7 des Kalibrierventils abgesperrt, und der Druckabbau im Druckraum 9 bewirkt die Freigabe des Sensorteilraums 4 zur Verbindung mit der Ventildurchgangsöffnung 5 dadurch, daß sich die Ventilplatte 13 unter der Einwirkung der Öffnungsvorspannungsfeder 14 in die Öffnungsendlage bewegt.

In beiden Endlagen ist der Durchlaß der Hauptströmung in der Ventildurchgangsöffnung 5 nahezu unbehindert.

**Ansprüche**

1. Kalibrierventil für Gassensoren, insbesondere für einen Sauerstoffsensor in der Atemgasführung eines Beatmungsgerätes, **dadurch gekennzeichnet,** daß in Verbindung mit der Ventildurchgangsöffnung (5) ein durch ein Ventilelement (12,13) absperrbarer mit einem Spülgas- bzw. Kalibriergaseinlaß (6) und -auslaß (7) versehener Sensorteilraum (4) vorgesehen ist und daß das Ventilelement (12,13) durch ein en Steuerantrieb (11) in zwei Endlagen bewegbar ist, wobei der Sensorteilraum (4) in der einen Endlage bei geschlossenem Spülgas- bzw. Kalibriergasein laß (6) und -auslaß (7) in Strömungsverbindung mit der Ventildurchgangsöffnung (5) steht, während er in der anderen Endlage durch das Ventilelement (12,13 von der Ventildurchgangsöffnung (5) abgesperrt ist.

2. Kalibrierventil nach Anspruch 1, **dadurch gekennzeichnet,** daß die Steuerung von Spulgas- bzw. Kalibriergaseinlaß (6) und -auslaß (7) mit dem Steuerantrieb (11) des Ventilelementes (12,13) gekoppelt ist.

3. Kalibrierventil nach Anspruch 1, **dadurch gekennzeichnet,** daß das Ventilelement (12,13) mit einer einen Druckraum (9) abschließenden Schaltmembran (11) verbunden ist.

4. Kalibrierventil nach Anspruch 1, **dadurch gekennzeichnet,** daß das Ventilelement (12,13) mit einem elektromagnetischen Steuerantrieb verbunden ist.

5. Kalibrierventil nach Anspruch 1 **dadurch gekennzeichnet,** daß der Sensorteilraum (4) durch einen seitlichen Ansatz (3) des Ventilgehäuses (2) gebildet wird.

6. Kalibrierventil nach Anspruch 5 **dadurch gekennzeichnet,** daß der seitliche Gehäuseansatz (3) ein Steckansatz zum Aufstecken des Sensors (16) ist.

7. Kalibrierventil nach Anspruch 1, **dadurch gekennzeichnet,** daß das Ventilelement (12,13) eine gegen eine Ringdichtung (15) des Gehäuses (2) anliegende Ventilplatte (13) ist, welche mit einer Öffnungsvorspannungsfeder (14) abgestützt ist.

8. Schaltungsanordnung zur Benutzung in Verbindung mit einem Kalibrierventil nach Anspruch 3, **dadurch gekennzeichnet,** daß ein 2/2 Wege-Magnetventil (18) vorgesehen ist, welches die Schaltmem bran (11) des Kalibrierventils (1) und gleichzeitig die Spülgaszuführung zum Zufluß von Spülgas in den Einlaß (6) des Sensorteilraums (4) schaltet, während der Auslaß (7) des Sensorteilraums (4) durch einen pneumatischen Schalter (21) nach der Atmosphäre hin geöffnet wird.

9. Schaltungsanordnung nach Anspruch 8, **durch gekennzeichnet,** daß in der Gaszuleitung zu Spül- bzw. Kalibriergaseinlaß (6) und in der Zuführungsleitung zum Druckraum (9) der Schaltmembran (11) durchflußbegrenzende Drosselstellen (23,24) eingeschaltet sind.

Fig. 1

Fig. 2

Fig. 3